Europäisches Patentamt

European Patent Office

Office européen des brevets

(19)

(11) Publication number: **0 289 036**

**A1**

(12) **EUROPEAN PATENT APPLICATION**

(21) Application number: **88106896.9**

(22) Date of filing: **29.04.88**

(51) Int. Cl.⁴: **C07C 25/13 , C07C 17/20**

(30) Priority: **30.04.87 US 44174**

(43) Date of publication of application:
**02.11.88 Bulletin 88/44**

(84) Designated Contracting States:
**BE DE FR GB IT NL**

(71) Applicant: **OCCIDENTAL CHEMICAL CORPORATION**
**P.O. Box 189**
**Niagara Falls New York 14302(US)**

(72) Inventor: **Mundhenke, Rudolph F.**
**1329 Master Street**
**North Tonawanda New York 14120(US)**
Inventor: **Tang, David Y.**
**36 Valleybrook Lane**
**East Amherst New York 14051(US)**

(74) Representative: **Vossius & Partner**
**Siebertstrasse 4 P.O. Box 86 07 67**
**D-8000 München 86(DE)**

(54) **Preparation of 3-chloro-4,5-difluorobenzotrifluoride.**

(57) In the synthesis of 3-chloro-4,5-difluorobenzotrifluoride by reaction of KF with 3,5-dichloro-4-fluorobenzotrifluoride, the production of undesirable by-products, especially over-fluorinated materials such as trifluorobenzotrifluoride is minimized when the reaction is run at a temperature of 196° to 206° Celsius in the presence of N-methyl-2-pyrrolidone.

EP 0 289 036 A1

# PREPARATION OF 3-CHLORO-4,5-DIFLUOROBENZOTRIFLUORIDE

This invention relates to a method for the preparation of 3-chloro-4,5-difluorobenzotrifluoride. The compound is useful as an intermediate in the preparation of various chemical products, in particular in the preparation of substituted diphenyl ethers having herbicidal properties.

Various methods for the preparation of 3-chloro-4,5-difluorobenzotrifluoride have been described in the literature. One such method, disclosed in U.S. Patent No. 4,388,472 to Cartwright et al, involves the steps of (1) reacting chlorine with p-trifluoromethylaniline in glacial acetic acid to form 2,6-dichloro-4-trifluoromethylaniline; (2) diazotization of the latter; (3) reaction of the diazo product with cuprous chloride in concentrated hydrochloric acid to form 3,4,5-trichlorobenzotrifluoride and (4) reaction with KF to form 3-chloro-4,5-difluorobenzotrifluoride.

Such methods, although useful for laboratory preparations are less suitable for commercial scale preparations. The starting material, p-trifluoromethylaniline, is expensive. Furthermore, the diazotization reaction is expensive and not readily adaptable to commercial scale. Furthermore, step 4 of the prior art route of synthesis, that is, the reaction of KF with 3,4,5-trichlorobenzotrifluoride, leads to the formation of an isomeric mixture of 3-chloro-4,5-difluorobenzotrifluoride and 4-chloro-3,5-difluorobenzotrifluoride, and thus requires additional separation procedures. U.S. Patent No. 4,384,135 to Cartwright et al discloses the reaction of 3,4,5-trichlorobenzotrifluoride with KF to form 3-chloro-4,5-difluorobenzotrifluoride. The patentees further disclose the use of 3-chloro-4,5-difluorobenzotrifluoride as an intermediate in the subsequent preparation of a fluorine-containing diphenyl ether herbicide. U.S. Patent No. 4,582,948 discloses a process for the preparation of 3-chloro-4,5-difluorobenzotrifluoride by the vapor phase chloro denitration of 3,4-difluoro-5-nitrobenzotrifluoride.

In U.S. Patent No. 4,590,315 to Maul et al there is disclosed a process for the preparation of 3-chloro-4,5-difluorobenzotrifluoride comprising the steps of (a) reacting an alkali metal fluoride with 4-chloro-3,5-dinitrobenzotrifluoride to form 4-fluoro-3,5-dinitrobenzotrifluoride; (b) chloro denitrating the 4-fluoro product of step (a) to form 3,5-dichloro-4-fluorobenzotrifluoride; and (c) reacting the 3,5-dichloro-4-fluorobenzotrifluoride compound prepared in step (b) with an alkali metal fluoride to form 3-chloro-4,5-difluorobenzotrifluoride. The patentees suggest that the fluorination reaction of step (c) may be carried out at a temperature of about 170° to about 270° Celsius and preferably about 200° to about 270° Celsius and in the presence of a solvent, such as N-methyl-2-pyrrolidone, sulfolane, N,N-dimethylformamide, N,N-dimethyl-acetamide, N,N-diethylformamide, dimethylsulfoxide, diethylsulfoxide, dipropylsulfoxide, dioctylsulfoxide, dimethylsulfone, diethylsulfone, diphenylsulfone, and the like, and mixtures thereof. The patentees recommend higher boiling solvents, such as sulfolane as preferred since it is preferred to carry out the reaction at higher temperatures. The reaction disclosed leads to the formation of substantial quantities of 3,4,5-trifluorobenzotrifluoride as an unwanted by-product.

Although prior art methods for the preparation of 3-chloro-4,5-difluorobenzotrifluoride are known and are commercially useful, such processes are characterized by the disadvantage of formation of unwanted by-products. The formation of such by-products, especially 3,4,5-trifluorobenzotrifluoride, results not only in an economic disadvantage with respect to efficient utilization of starting materials and the necessity of added separation steps to remove such by-products, but also an additional disposal problem dictated by present day environmental considerations. Those skilled in the art will appreciate the need for a process that approaches zero environmental discharge.

Accordingly, it is an object of this invention to provide a process for the preparation of high purity 3-chloro-4,5-difluorobenzotrifluoride that is well-suited for both laboratory and commercial scale preparations in that it is characterized by minimal production of undesired by-products, especially trifluorobenzotrifluoride, and avoidance of the attendant environmental problems.

It has now been found that, in the synthesis of 3-chloro-4,5-difluorobenzotrifluoride by reaction of potassium fluoride with 3,5-dichloro-4-fluorobenzotrifluoride, the production of undesirable by-products, especially over-fluorinated materials such as trifluorobenzotrifluoride is minimized when the reaction is run at a temperature of about 196° to about 206° Celsius in the presence of N-methyl-2-pyrrolidone.

The proportions of reactants may vary considerably. It is preferred however, to provide a stoichiometric excess of potassium fluoride and most preferably a molar ratio of about 2:1 to about 10:1 of potassium fluoride:fluoro-dichlorobenzotrifluoride.

It has been found that the halogenation exchange that forms the basis of the process of the invention may be accelerated by the addition of cesium fluoride to the reaction mixture. The amount of cesium fluoride that may be incorporated in the reaction mixture may vary considerably, but will typically be in the range of about 0.1 percent to about 10 percent by weight based on the weight of KF.

The process of the invention may be efficiently operated on a continuous basis with reaction product being removed by distillation and starting material added slowly (in addition to starting material recycled and/or returned by reflux) to compensate for that consumed in the reaction.

The process is preferably carried out under reflux conditions at atmospheric pressure. Product distillate is preferably removed at a vapor temperature of between the boiling points of the starting 3,5-dichloro-4-fluorobenzotrifluoride (b.p. 166° Celsius) and that of the product 3-chloro-4,5-difluorobenzotrifluoride (b.p. 131-132° Celsius) to preferentially remove the 3-chloro-4,5-difluorobenzotrifluoride and any by-product 3,4,5-trifluorobenzotrifluoride (b.p. 98° Celsius). It is most preferred to remove the distillate at a vapor temperature of about 148° to about 153° Celsius.

When the preparation of 3-chloro-4,5-difluorobenzotrifluoride by reaction of potassium fluoride with 3,5-dichloro-4-fluorobenzotrifluoride was carried out in accordance with the parameters set forth above, that is, in the presence of N-methyl-2-pyrrolidone and at a reaction temperature of about 196° to about 206° Celsius, a product distillate containing 57.5 percent of the desired 3-chloro-4,5-difluorobenzotrifluoride and 0.26 percent 3,4,5-trifluorobenzotrifluoride (GLC analysis, area percent) was produced. By contrast, when the reaction was run in sulfolane, at a temperature of about 192° to 216° Celsius, a product distillate containing 53-60 percent 3-chloro-4,5-difluorobenzotrifluoride and 5-10 percent 3,4,5-trifluorobenzotrifluoride. Carrying out the reaction in N-methyl pyrrolidone at a temperature of 188° to 190° Celsius resulted in a product distillate containing 88-89 percent 3-chloro-4,5-difluorobenzotrifluoride and 2-5 percent 3,4,5-trifluorobenzotrifluoride. Thus, it will be seen that the preparations of 3-chloro-4,5-difluorobenzotrifluoride in accordance with the present invention, is especially advantageous in minimizing the production of undesired over-fluorinated materials and avoidance of the attendant disposal problems.

The following specific examples are provided to further illustrate this invention and the manner in which it may be carried out. It will be understood, however, that the specific details given in the examples have been chosen for purpose of illustration and are not to be construed as a limitation on the invention. In the examples, unless otherwise indicated, all parts and percentages are by weight and all temperatures are in degrees Celsius.

Example 1

A mixture of 161 parts of anhydrous potassium fluoride in 671 parts of n-methyl pyrrolidone was heated to about 196°C and 94.4 parts of 3,5-dichloro-4-fluorobenzotrifluoride was added. An additional 155.6 parts of 3,5-dichloro-4-fluorobenzotrifluoride was added slowly over a reaction period of 34.5 hours during which the temperature of the reaction mixture was 196°-202°C. After 23.5 hours of reaction time, 4.2 parts of CsF was added to the reaction mixture. During the reaction, temperature of the refluxing vapors was maintained at 150°-151°C and a total of 168.2 parts of distillate was removed in periodic sampling and analyzed by gas chromatographic methods with the results shown in Table 1.

3

0 289 036

## Table 1

| Sample | Reaction Time (hrs) | Sample Weight (parts) | Product[1] | | |
| | | | CDFBTF[2] (parts) | TFBTF[3] (parts) | CDFBTF[2] / TFBTF[3] |
|---|---|---|---|---|---|
| 1 | 10.0 | 34.4 | 17.1 | 0.06 | 256/1 |
| 2 | 23.5 | 39.2 | 21.9 | 0.03 | 559/1 |
| 3 | 27.0 | 31.2 | 17.9 | 0.09 | 192/1 |
| 4 | 31.0 | 34.1 | 20.9 | 0.1 | 204/1 |
| 5 | 34.5 | 30.3 | 18.1 | 0.1 | 199/1 |

1) Based on GLC area percent

2) 3-chloro-4,5-difluorobenzotrifluoride

3) 3,4,5-trifluorobenzotrifluoride

**Claims**

1. A process for the preparation of 3-chloro-4,5-difluorobenzotrifluoride which comprises reacting 3,5-dichloro-4-fluorobenzotrifluoride with potassium fluoride in the presence of N-methyl-2-pyrrolidone at a temperature of about 196° to about 206° Celsius.

2. A process according to Claim 1 carried out in the presence of cesium fluoride.

3. A process according to Claim 1 wherein the molar ratio of potassium fluoride:3,5-dichloro-4-fluorobenzotrifluoride is about 2:1 to about 10:1.

4. A process according to Claim 1 carried out under reflux conditions.

5. A process according to Claim 4 wherein the product is removed by distillation at a vapor temperature of about 131° to 166° Celsius.

6. A process according to Claim 4 wherein the produce is removed by distillation at a vapor temperature of about 148° to about 153° Celsius.

4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US-A-4 590 315  (MAUL et al.)<br>* Column 2, lines 21-63; claim 10C * | 1 | C 07 C   25/13<br>C 07 C   17/20 |

TECHNICAL FIELDS
SEARCHED (Int. Cl.4)

C 07 C   25/00
C 07 C   17/00

The present search report has been drawn up for all claims

| Place of search | Date of completion of the search | Examiner |
|---|---|---|
| THE HAGUE | 11-07-1988 | VAN GEYT J.J.A. |